# EUROPEAN PATENT SPECIFICATION

(11) **EP 1 496 990 B1**
(45) Date of publication and mention of the grant of the patent: **01.12.2010**
(21) Application number: 03723074.5
(22) Date of filing: 18.04.2003
(51) Int. Cl.: A61N 5/00, A61N 1/06, A61N 5/04, A61B 18/14

(54) **INSTRUMENT WITH ACTIVE RADIO-FREQUENCY WIRES FOR TREATMENT OF TUMOURS**
GERÄT MIT AKTIVEN RADIOFREQUENZ-DRÄHTEN ZUR TUMORBEHANDLUNG
INSTRUMENT COMPORTANT DES FILS A FREQUENCE RADIO ACTIVE POUR TRAITEMENT DE TUMEURS

(30) Priority: 23.04.2002 IT BS20020046 U
(43) Date of publication of application: 19.01.2005
(73) Proprietor: FOGAZZI DI VENTURELLI ANDREA & C. S.N.C., 25062 Concesio (Brescia) (IT)
(72) Inventor: VENTURELLI, Andrea, I-25062 Concesio,BRESCIA (IT)
(74) Representative: Crippa, Paolo Ernesto
(86) International application number: PCT/IT2003/000250
(87) International publication number: WO 2003/090864

(56) References cited:
- EP-A- 0 861 676
- US-A- 5 800 494
- US-A- 5 993 462

## Description

### Field of the Invention

This invention concerns an instrument in the form of a needle-electrode for radio-frequency induced hyperthermia treatment of tumours.

### State of the Art

Catheter - sounds with at least one terminal electrode or needles with a straight radio-frequency active wire are already available in the treatment of tumoral masses by means of radio frequency induced hyperthermia.

However, the catheter - sounds are relatively large and, even if efficient because of the presence of an important electrode, they have a drawback in that they can only be inserted into the body of the patient until they come into contact with the tumoral mass through natural patent ducts or made patent by dilation.

The needles with an active wire can be introduced into the body by inserting them into the tissues, but their action is limited due to the small diameter of the active wire, which can only influence limited areas and therefore results in longer treatment periods and times. For treatment of larger tumoral masses, larger needles are used, and their use becomes more traumatic for the patient.

In order to improve the efficacy of a radio-frequency active electrode needle in the treatment of very large tumoral masses, keeping nevertheless the diameter within limited dimensions in order to reduce the traumatic effect, a medicinal instrument was projected which includes a hollow guide needle traversed by a single radio-frequency active wire having a terminal segment which is rectilinear when retracted in the needle and becomes helicoidal or spiral when it is in the forward position, emerging from the end of the needle.

In this way, the active wire even if thin is able to irradiate and therefore treat a larger surrounding area in proportion to the area of its cross section.

US 5 672 174, EP 0 861 676 and US 5 431 649 disclose instruments similar to the one set forth in the preamble of claim 1

### Objective and Summary of the Invention

These radio-frequency active electrode needles are however open to further improvement. One objective of this invention is in fact to supply an electrode needle for hyperthermia treatment of tumours achieved by a new, original combination of the elements having improved functional efficiency and efficacy.

This objective is achieved with a hollow guide needle housing three radio-frequency active wires, where said wires can be moved either together or singularly between an inactive retracted position in the guide needle and an active protruding position from a distal end of said needle, and where the wires each have a proximal end which can be connected to a radio frequency generator and a pre-shaped distal part to move from a substantially rectilinear form, when the wire is in the retracted position, to a helicoidal or spiral shape, when the wire is in the active protruding position from the guide needle.

The three wires form in this way an equivalent number of electrodes which contemporaneously or selectively activated and/or oriented according to needs enable treatment of vast tumoral areas increasing the efficacy of the instrument and reducing intervention times.

### Brief Description of the Drawings

Greater details of the invention will become evident from the following description made with reference to the indicative and non-limiting drawings enclosed, in which:
Fig. 1 shows a needle with two wires in the forward position;
Fig. 2 shows an enlarged longitudinal cross-section of a distal part of the needle in Fig. 1 with wires retracted; which is not part of the invention
Fig. 3 shows a cross-section of the needle in Fig. 1 enlarged even further; which is not part of the invention
Fig. 4 shows a cross-section of an instrument with wires housed in respective ducts in the guide needle;
Fig. 5 shows an instrument with a wire winding device for moving them in the guide needle; and
Fig. 6 shows a cross section of a variation in construction of the needle.

### Detailed Description of the Invention

As shown, the medical instrument includes a guide needle 11 equipped with a grip 12 and destined to be inserted into the body of the patient until it reaches the tumoral area to be treated.

The guide needle 11 can be made of a tubular element having a longitudinal hole 13 with three wires extending from the proximal to the distal part 14, 15 .., movable in lengthwise between a fully retracted position of the needle and a forward protruding position of the distal end of the needle itself.

Externally, the guide needle 11 has an insulating sheath 11' along all its length except for the distal end section 11 ".

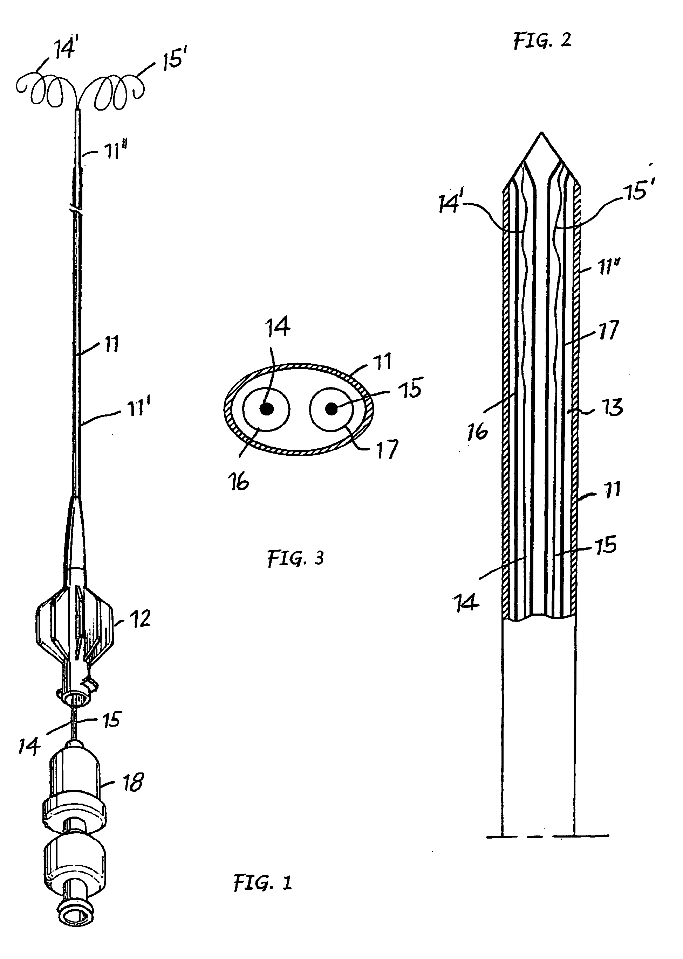

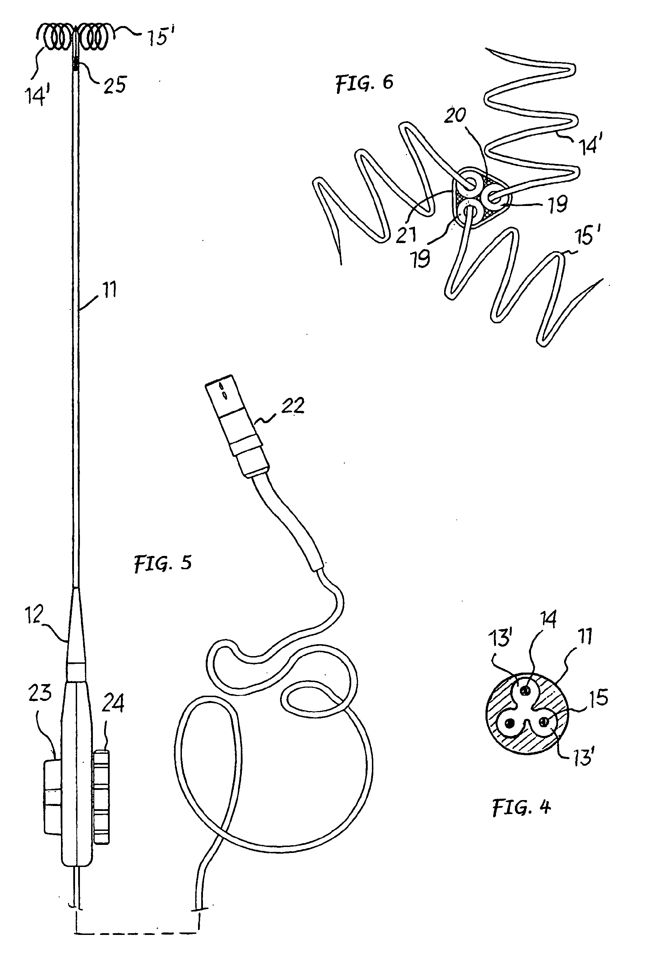

The wires 14, 15 can each be guided in a separate duct 16, 17, placed inside the guide needle, as shown in Figs. 2 and 3. Otherwise the wires 15, 15 can be freely housed in the longitudinal hole 13 of the guide needle 11. Differently again, as shown in Fig. 4, they can each be housed individually in separate ducts 13' provided in said hole 13 of the guide needle 11.

In one of the variations, the instrument can have a needle made up of the same number of tubes 19 as the active wires 14, 15 .... With a wire in each tube. The tubes 19 are placed side by side and welded together using a resin or something similar 20 and enclosed in an insulating sheath 21, as shown in Fig. 6.

Wires 14, 15 are practically straight except for a distal section 14', 15' having respectively, either a helicoidal or spiral configuration, prepared during a pre-shaping process.

The proximal ends of the wires can all be attached to a connection 18 and connected by means of a connector 22 - Fig. 5 - to a radio-frequency generator - not shown. In this way wires 14, 15 can be moved forwards or backwards at the same time in the needle guide 11 or in the respective tubes 16, 17, 19 between a passive position where they are fully retracted inside the needle guide as shown in Fig. 2, and an active position where their terminal segments 14', 15' protrude from the distal end of the needle itself having a helicoidal or spiral configuration as shown in Fig. 1 and 5.

As an alternative, each wire 14, 15,... can be attached to its own connection and connected, by the latter, to a radio-frequency generator. In this case and according to needs, the wires, each being independent of the other, can be manoeuvred and moved singularly, or together, between the passive and active positions.

Furthermore, the longitudinal movements of the wires between said passive and active positions, may be achieved by means of a winder pulley 23 - Fig. 5 - to which the proximal ends of the wires are connected and which can be turned using a handle 24 associated with and needle indicating the longitudinal position of the wires.

The helicoidal or spiral segments 14', 15' of wires 14, 15 can be oriented in different directions when, protruding from the needle they are in the active position. Orienting the wires can, in addition, be changed by turning the connection or connections the wires, as the case may be, are connected to.

Worthy of note is the fact that the distal end of the guide needle can also be equipped with a thermocouple 25 to read the temperature in the area to be treated with the instrument described herein.

While the needle is being introduced into the body of the patient, the wires 14, 15,.. are held in the passive position, retracted in the needle. Their distal segments 14', 15'... remain as it were almost rectilinear, constrained and housed in the needle cavity. When the needle reaches the area to be treated, the distal segments of the wires are made to protrude from the distal end of the needle and automatically become helicoidal or spiral. In this way, once connected to the radio-frequency generator they irradiate the area to be treated, necrotising the ill tissue using hyperthermia induced by radio-frequency.

## Claims

1. Medical instrument for the treatment of tumours by radio-frequency induced hyperthermia, comprising a guide needle (11) having a proximal part with a grip and a distal part, said needle (11) being introduceable into a body by inserting it into tissues; and three active radio-frequency wires (14, 15) which run lengthwise in said guide needle and which are connected at their proximal end to a radio-frequency generator each one having either a helicoidal or spiral distal segment (14', 15'), said wires (14,15) being movable longitudinally between a completely retracted into the needle passive position and an active position where the distal segments (14', 15') of the wires protrude from the distal end of the guide needle, automatically adopting a coiled shape, **characterized in that** the guide needle (11) is made up of three small tubes (19) placed side by side with each tube (19) in contact with the near one, welded to each other and housed in an insulating sheath (21) avoiding to leave space between adjacent tubes(19) and the tubes (19 and the sheath (21), said insulating sheath housing the three tubes except for their distal end, and said wires (14, 15) are individually placed, each one occupying one of said small tubes.

2. Medical instrument according to claim 1, wherein the guide needle (11) is made up of a tubular element and said wires (14,15) pass freely in the guide needle.

3. Medical instrument according to claim 1, wherein the guide needle (11) is made up of a tubular element and said wires (14,15) are housed longitudinally in grooves provided in said needle.

4. Medical instrument according to claim 1, wherein the guide needle (11) is made up of a tubular element and said wires (14,15) are individually housed in guide tubes (16,17) placed in the guide needle.

## Patentansprüche

1. Medizinisches Instrument für die Behandlung von Tumoren durch hochfrequenzinduzierte Hyperthermie, enthaltend eine Führungsnadel (11), die einen proximalen Teil mit einem Griff und einen distalen Teil hat, wobei die Nadel (11) in einen Körper einführbar ist, indem sie in Gewebe eingeführt wird; sowie drei aktive Hochfrequenzdrähte (14, 15), die in Längsrichtung in der Führungsnadel verlaufen und an ihrem proximalen Ende mit einem Hochfrequenzgenerator verbunden sind und jeweils entweder über ein schraubenförmiges oder spiralförmiges distales Segment (14', 15') verfügen, wobei diese Drähte (14, 15) in Längsrichtung zwischen einer vollständig in die Nadel zurückgezogenen, passiven Stellung und einer aktiven Stellung beweglich sind, in der die distalen Segmente (14', 15') der Drähte von dem distalen Ende der Führungsnadel hervorragen und automatisch eine gewundene Form annehmen, **dadurch gekennzeichnet, dass** die Führungsnadel (11) aus drei kleinen Röhren (19) besteht, die nebeneinander angeordnet sind, wobei jede Röhre (19) die nächstgelegene berührt, diese miteinander verschweißt und in einer Isolierumhüllung (21) aufgenommen sind, wobei es vermieden ist, dass ein Zwischenraum zwischen benachbarten Röhren (19) sowie den Röhren (19) und der Isolierumhüllung (21) zurückbleibt, die Isolierumhüllung die drei Röhren mit Ausnahme ihrer distalen Enden aufnimmt und die Drähte (14, 15) derart einzeln angeordnet sind, dass jeder einzelne eine der kleinen Röhren einnimmt.

2. Medizinisches Instrument nach Anspruch 1, bei dem die Führungsnadel (11) aus einem röhrenförmigen Element besteht und die Drähte (14, 15) die Führungsnadel frei durchlaufen.

3. Medizinisches Instrument nach Anspruch 1, bei dem die Führungsnadel (11) aus einem röhrenförmigen Element besteht und die Drähte (14, 15) in Längsrichtung in Rillen aufgenommen sind, die in der Nadel vorgesehen sind.

4. Medizinisches Instrument nach Anspruch 1, bei dem die Führungsnadel (11) aus einem röhrenförmigen Element besteht und die Drähte (14, 15) einzeln in Führungsröhren (16, 17) aufgenommen sind, die sich in der Führungsnadel befinden.

## Revendications

1. Instrument médical pour le traitement de tumeurs par hyperthermie induite par radiofréquences, comprenant une aiguille de guidage (11) ayant une partie proximale avec une poignée et une partie distale, ladite aiguille (11) pouvant être introduite dans un corps en l'insérant dans des tissus ; et trois fils à radiofréquence active (14, 15) qui circulent longitudinalement dans ladite aiguille de guidage et qui sont reliés à leur extrémité proximale à un générateur de radiofréquences, chacun ayant soit un segment distal hélicoïdal ou spiralé (14', 15'), lesdits fils (14, 15) étant mobiles longitudinalement entre une position passive complètement rétractée dans l'aiguille et une position active où les segments distaux (14', 15') des fils dépassent de l'extrémité distale de l'aiguille de guidage, adoptant automatiquement une forme spiralée, **caractérisé en ce que** l'aiguille de guidage (11) est faite de trois petits tubes (19) placés côte à côte, chaque tube (19) étant en contact avec le tube à proximité, soudés les uns aux autres et logés dans une gaine isolante (21) ce qui évite de laisser un espace entre des tubes adjacents (19) et les tubes (19) et la gaine (21), ladite gaine isolante logeant les trois tubes à l'exception de leur extrémité distale, et lesdits fils (14, 15) sont placés individuellement, chacun d'eux occupant l'un desdits petits tubes.

2. Instrument médical selon la revendication 1, où l'aiguille de guidage (11) est faite d'un élément tubulaire et lesdits fils (14, 15) passent librement dans l'aiguille de guidage.

3. Instrument médical selon la revendication 1, où l'aiguille de guidage (11) est faite d'un élément tubulaire et lesdits fils (14, 15) sont logés longitudinalement dans des rainures disposées dans ladite aiguille.

4. Instrument médical selon la revendication 1, où l'aiguille de guidage (11) est faite d'un élément tubulaire et lesdits fils (14, 15) sont logés individuellement dans des tubes de guidage (16, 17) placés dans l'aiguille de guidage.
